# FASCICULE DE BREVET EUROPEEN

(11) **EP 2 265 132 B1**
(45) Date de publication et mention de la délivrance du brevet: **10.06.2015**
(21) Numéro de dépôt: 09742255.4
(22) Date de dépôt: 07.04.2009
(51) Int. Cl.: A23K 1/18, A01K 67/033

(54) **PRODUCTION DE "MINI-LARVES" VIVANTES D'INSECTES ET LEUR UTILISATION POUR L'ALIMENTATION DES POISSONS D'AQUARIUM, DES ALEVINS DES POISSONS D'ÉLEVAGE ET DES ANIMAUX DE COMPAGNIE**
HERSTELLUNG LEBENDER MINI-INSEKTENLARVEN UND IHRE VERWENDUNG ZUR FÜTTERUNG VON AQUARIUMSFISCHEN, ZUCHTFISCHLARVEN UND HAUSTIEREN
PRODUCTION OF LIVE INSECT MINI-LARVAE AND USE THEREOF FOR FEEDING AQUARIUM FISH, ALEVINS OF FARM FISH AND PETS

(30) Priorité: 16.04.2008 FR 0802096
(43) Date de publication de la demande: 29.12.2010
(73) Titulaire: Institut de Recherche pour le Développement ( IRD), 13572 Marseille Cédex 02 (FR); Agency For Marine And Fisherie Research And Development, The Ministry of Marine Affairs and Fisheries, the Republic of Indonesia, Jakarta 14430 (ID)
(72) Inventeur: HEM, Saurin, F-34000 Montpellier (FR); FAHMI, Melta Rini, Depok 16436 (ID)
(74) Mandataire: Grosset-Fournier, Chantal Catherine
(86) Numéro de dépôt international: PCT/FR2009/050592
(87) Numéro de publication internationale: WO 2009/136057

(56) Documents cités:
- EP-A- 1 380 206
- WO-A-95/26633
- DE-A1- 10 328 102
- FR-A- 2 868 911

## Description

La présente invention a pour objet la production de « mini-larves » vivantes d'insectes et leur utilisation pour l'alimentation des poissons d'aquarium, des alevins des poissons d'élevage ainsi que comme appâts ou nourriture pour animaux de compagnie ou de laboratoires comme les petits rongeurs (souris, rats...), les lézards et les oiseaux.

Au cours des dernières décennies, l'aquariophilie a connu un développement très rapide et il existe un engouement considérable du grand public pour des aquariums qui, sans cesse, s'enrichissent de diverses espèces animales maintenues dans des écosystèmes artificiels toujours plus complexes et proches des conditions du milieu naturel dans lequel se trouvaient ces espèces animales.

Il est maintenant devenu rare qu'une grande ville ne soit pas dotée de son aquarium public qui constitue à l'évidence un lieu pédagogique privilégié de découverte, accessible à tous et, bien souvent mis au service des scientifiques qui peuvent ainsi y étudier aisément telles ou telles espèces animales aquatiques. De ce point de vue, compte tenu du contexte actuel dans lequel les préoccupations d'ordre environnemental deviennent chaque jour un peu plus marquées, ces aquariums constituent des outils extrêmement importants pour aider à la compréhension, à la défense et à la préservation des milieux marin et aquatique.

Par ailleurs, la raréfaction de certaines espèces de poissons marins ou d'eau douce qui pendant des décennies ont fait l'objet de pêches intensives, soulève d'importantes questions écologiques et économiques qui conduisent actuellement au développement de fermes et d'élevages aquacoles de plus en plus nombreux et sophistiqués.

Le développement simultané des marchés de l'aquariophilie et de la pisciculture engendre des besoins croissants en aliments pour poissons qui doivent bien entendu se diversifier et s'adapter à cette demande. Actuellement, ces aliments se présentent, pour l'essentiel, sous la forme de farines, de granulés ou de paillettes qui sont préservés dans des emballages stériles et qui sont, en principe, formulés pour pouvoir répondre aux besoins nutritionnels des poissons maintenus en captivité.

Il apparaît néanmoins que ces aliments présentent un certain nombre d'inconvénients. En particulier, il a été observé que certaines espèces de poissons refusant tout simplement de les consommer ou ne parvenant pas à les absorber du fait de la trop grande granulométrie des particules de nourriture, ces aliments s'accumulent dans les bassins où ils contribuent grandement à la dégradation de la qualité des eaux. Au-delà de son impact négatif sur la croissance et le développement des poissons et alevins, cette situation engendre un surcroît de travail d'entretien et de maintenance des aquariums et des bassins d'élevage qui, hormis les contraintes qu'ils génèrent, sont à l'origine de surcoûts non négligeables. De plus, l'approvisionnement en ce type d'aliments ne parvenant plus à satisfaire la demande croissante, une forte augmentation de leur prix a pu être constatée.

Progressivement, le marché des aliments constitués de proies vivantes, telles que les larves de chironomes ou « vers de vase » (diptères), les micro-vers (nématodes) les lombrics, etc.., perçues comme plus naturelles et beaucoup plus attrayantes pour les poissons, se développe. Toutefois, l'inconvénient lié à l'utilisation de ce type d'alimentation réside dans le risque de dissémination dans les aquariums et dans les bassins d'élevage de germes pathogènes dont il est porteur.

Les animaux de compagnie, comme les petits rongeurs, les lézards et les oiseaux nécessitent également une alimentation exempte de germes pathogènes et qui soit facile à ingérer. Les larves d'insectes sont largement utilisées, notamment celles de Hermetia illucens.

D'autres utilisations de larves d'insectes ont été décrites dans l'art antérieur. Le document DE10328102 A1 divulgue un procédé de production de larves et de pupes pour promouvoir la cicatrisation. Dans le document FR 2 868 911, les larves d'insectes sont utilisées pour la dégradation des matières riches en protéines et/ou en lipides, avec application à la biodégradation de tourteaux de palmiste et à la reconversion en biomasse d'asticots.

D'une manière inattendue, l'inventeur a observé qu'il était possible d'obtenir des larves de petite taille (i.e. «mini-larves ») disposant d'une morphologie tout à fait identique à celle des larves de taille normale et avec des rendements pondéraux identiques en terme de biomasse.

La présente invention a pour but de fournir un procédé de production de mini-larves d'insectes vivantes constituant un aliment attrayant pour les poissons et alevins, notamment les plus petits d'entre eux ainsi que pour les animaux de compagnie. Ces proies vivantes réputées saines et dépourvues de tous germe pathogène seront consommées de manière optimale par les poissons, les animaux de laboratoire ou les animaux de compagnie et, par conséquent, éviteront le gaspillage, la pollution des eaux et les pertes financières qu'ils entraînent.

L'expression « mini-larves » désigne des larves vivantes d'insectes dont le diamètre corporel n'excède pas 4mm, voire 3 mm et la longueur n'excède pas 10 mm

L'expression « les plus petits d'entre eux » fait référence à des poissons et alevins dont le diamètre de la bouche n'excède pas 4 mm.

L'expression « proies vivantes » désigne les organismes vivants destinés à l'alimentation des poissons et devant :
- créer une bonne appétence des poissons
- comporter les qualités nutritives requises,
- être facilement disponibles et prêts à répondre aux demandes du marché,
- être produits en masse selon des techniques susceptibles d'être industrialisées et économiquement viables,
- être dotés de caractéristiques physiologiques les rendant capables de survivre à leurs conditions de conservation sans que cela n'entraîne de perte de leur qualité nutritive,
- être conservés simplement (e.g. température ambiante ou réfrigérateur),
- ne pas dégager d'odeur désagréable,
- être propres et non-porteurs de germes pathogènes.

La présente invention a donc pour objet, l'utilisation d'un milieu de culture comprenant un mélange d'eau, de céréales et/ou de tourteaux et, éventuellement, un agent de fermentation ajouté pour l'incubation d'oeufs d'insectes en vue de l'obtention de larves conservées vivantes, dont le diamètre corporel maximal de 1 à 4 mm, notamment de 2 mm environ et dont la longueur corporelle maximale de 4 à 12, notamment de 5 à 12 mm, notamment de 4 à 8 mm, notamment de 9 mm, notamment de 6 mm sont maintenus sensiblement constants pendant au moins 4 semaines, notamment 6 semaines.

Dans un mode de réalisation avantageux de l'invention les céréales et/ou les tourteaux du milieu de culture sont soumis à une fermentation, à l'obscurité ou à l'air libre, pendant 1 semaine, à une température de 27 à 40°C, notamment de 27 à 32°C.

Dans un autre mode de réalisation avantageux de l'invention, la proportion pondérale eau/céréales ou eau/tourteaux est de 1 à 3, notamment de 1 à 2,2 et, est, de préférence, égale à 2.

Dans un autre mode de réalisation avantageux de l'invention, la proportion eau/céréales + tourteaux est de 1 à 3, de préférence égale à 2.

Selon l'invention l'agent de fermentation ajouté est présent à raison de 0 à 2% en poids par rapport au poids sec de céréales et/ou de tourteaux.

Dans un mode de réalisation particulièrement avantageux, le milieu de culture utilisé selon l'invention comprend des tourteaux et est totalement dépourvu de tout agent de fermentation ajouté.

Selon l'invention, l'agent de fermentation est choisi parmi les bactéries, les champignons microscopiques, notamment, les levures ; les céréales du milieu de culture sont choisies parmi le blé, l'épeautre, le maïs, l'orge, l'escourgeon, le riz, le millet, le seigle, le sorgho et l'avoine. L'avoine étant particulièrement utilisée et les tourteaux du milieu de culture sont choisis parmi les tourteaux de soja, d'arachide, de colza, de tournesol, de lin, de pépin de raisin, d'olive, de copra et de palmiste, le tourteau de palmiste étant particulièrement utilisé.

L'invention a également pour objet, une composition comprenant :
- un milieu de culture, lequel comprend un mélange d'eau, de céréales et/ou de tourteaux et, éventuellement, un agent de fermentation et,
- des oeufs et/ou des larves d'insectes incubés dans ledit milieu de culture.

Dans un mode de réalisation avantageux de l'invention, la composition comprend des oeufs d'insectes incubés à raison de 0,5 à 1,5 g/kg de milieu de culture, notamment 1 g/kg de milieu de culture.

Avantageusement les larves d'insectes contenues dans la composition selon l'invention ont un diamètre corporel maximal de 1 à 4 mm, notamment de 2 mm et une longueur corporelle maximale notamment de 5 à 12 mm, notamment de 4 à 8 mm, notamment de 9 mm, notamment de 6 mm.

Dans un mode de réalisation encore plus avantageux, la composition comprend un milieu de culture, lequel comprend un mélange d'eau, de céréales et/ou de tourteaux et, éventuellement, un agent de fermentation, des oeufs incubés dans ledit milieu de culture à raison de 0,5 à 1,5 g/kg de milieu de culture, notamment 1 g/kg de milieu de culture, les céréales et/ou les tourteaux ayant été soumis à une fermentation, à l'obscurité ou à l'air libre, pendant 1 semaine et à une température de 27 à 40°C, notamment de 27 à 32°C, ladite composition comprenant également des larves d'insectes incubés dans ledit milieu de culture ayant un diamètre corporel maximal de 1 à 4 mm, notamment de 2 mm et une longueur corporelle maximale de 4 à 12 mm, notamment de 5 à 12 mm, notamment de 4 à 8 mm, notamment de 9 mm, notamment de 6 mm.

Dans un mode de réalisation avantageux de l'invention, la composition comprend des oeufs de *Hermetia illucens.* En effet il a été constaté que les mini-larves de *Hermetia illucens* obtenues par le procédé selon l'invention satisfont à l'ensemble des critères énoncés précédemment.

Dans le cas où on utilise le tourteau de palmiste comme milieu de culture pour *Hermetia illucens,* la proportion entre la masse d'oeufs d'insectes et la quantité de milieu de culture est égal à 500, avantageusement 1000, avantageusement 1500 encore plus avantageusement 2000.

L'invention a également pour objet un procédé d'obtention de larves d'insectes conservées vivantes, dont le diamètre maximal de 1 à 4 mm, notamment de 2 mm environ et dont la longueur corporelle maximale de 5 à 12 mm, notamment de 4 à 8 mm, notamment de 9 mm, notamment de 6 mm sont maintenus sensiblement constants pendant au moins 4 semaines, notamment 6 semaines, ledit procédé comprenant les étapes suivantes :
1) incubation d'oeufs d'insectes dans un milieu de culture comprenant un mélange d'eau, de céréales et/ou de tourteaux et, éventuellement, d'un agent de fermentation, ladite incubation étant conduite jusqu'à l'éclosion des oeufs et l'obtention des larves, pour obtenir une composition selon l'invention,
2) déshydratation de la susdite composition jusqu'à obtenir une teneur en eau inférieure à 15% (poids/poids), avantageusement de 10% (poids/poids),
3) collecte, à partir de la susdite composition, des larves d'insectes vivantes, de diamètre corporel maximal de 1 à 4 mm, notamment de 2 mm environ et de longueur corporelle maximale de 4 à 12 mm, notamment de 5 à 12 mm, notamment de 4 à 8 mm, notamment de 9 mm, notamment de 6 mm et
4) transfert des susdites larves vivantes collectées dans un environnement dépourvu de nourriture où elles sont maintenues vivantes, à l'obscurité, pendant au moins 4 semaines, notamment 6 semaines, sous un taux d'hygrométrie de 60 à 80% et à une température de 12 à 18°C, notamment 15°C.

Selon un mode de réalisation avantageux de la présente invention, la production de masse des mini-larves, comme proies vivantes, s'effectue, à partir d'oeufs fécondés, dans un espace clos et contrôlé dans lequel les insectes sont élevés et protégés. Cette méthode permet d'obtenir parfaitement et de manière homogène la croissance et le développement des larves et est donc la mieux adaptée à la production de masse des mini-larves. Les populations d'insectes choisies sont confinées dans une volière les isolant totalement de l'extérieur et les protégeant des prédations par d'autres insectes ou par des animaux tels que les rongeurs, les reptiles, les batraciens ou les oiseaux. Seuls les insectes de l'espèce choisie pour son élevage et la production des mini-larves peuvent se reproduire au sein de cette volière qui est aménagée de telle manière à reproduire autant que possible l'habitat naturel des insectes qui y sont maintenus.

Dans un mode de réalisation avantageux de la présente invention, l'élevage concerne des insectes du groupe des holométaboles en particulier, *Hermetia illucens.* Cet insecte est élevé à l'intérieur d'une volière dans une atmosphère dont le degré d'hygrométrie et la température sont maintenus respectivement de 70 à 80% et de 27 et 30°C. De manière avantageuse, des végétaux du type *Sphagneticola sp* peuplent la volière et favorisent le développement des insectes *Hermetia illucens.*

Les pupes d'insectes sont placées à l'intérieur de la volière dans des bacs d'exuviation en plastique (environ 5 000 à 10 000 pupes par bac en plastique). Après 2 à 5 semaines et après l'exuviation (*i.e.* mue imaginale dont est issue l'imago), les premiers insectes adultes apparaissent et, après 1 à 2 heures d'immobilité, s'envolent dans la volière pour s'y nourrir et s'y reproduire.

Selon un aspect particulièrement avantageux de la présente invention, les pupes sélectionnées sont des pupes de *Hermetia illucens* qui, après exuviation, se nourrissent de miel dilué dans de l'eau. Une semaine après l'exuviation mâles et femelles peuvent s'accoupler. Une semaine après l'accouplement, la femelle *Hermetia illucens* pond entre 400 et 1 200 oeufs.

Les oeufs d'insectes sont pondus préférentiellement dans des anfractuosités artificielles spécialement créées à cet effet à proximité immédiate d'un milieu de culture.

L'expression « anfractuosités artificielles » désigne, selon un aspect préféré de la présente invention, les plis et fronces générés sur le pourtour de l'ouverture d'un sac plastique, type « sac poubelle sans fond », muni de liens de fermeture intégrés, lorsque l'on tire sur lesdits liens pour fermer partiellement ledit sac lorsque celui-ci a été placé à proximité de milieu de culture.

Selon un aspect avantageux de la présente invention, la ponte préférentielle au niveau des susdites anfractuosités artificielles permet une collecte rapide et aisée de quantités importantes d'oeufs d'insectes. Il suffit en effet de récupérer les susdits sacs plastiques, en défronçant les plis et ouvrant les fronces et libérer les quantités importantes d'oeufs qu'elles contenaient et qui peuvent alors être aisément collectés.

L'expression « importantes quantité d'oeufs » désigne des récoltes qui, tous les 3 jours et pour l'ensemble de la volière, peuvent atteindre 15 à 20 g de biomasse d'oeufs, sachant que 1 g d'une telle biomasse contient environ 38 000 à 40 000 oeufs d'insectes.

Dans un mode de réalisation avantageux du procédé de l'invention, l'étape d'incubation et d'éclosion des oeufs d'insectes dans le milieu de culture est effectuée pendant 1 à 3 jours, notamment 2 jours, à une température de 24 à 35°C, notamment de 27 à 32°C, idéalement de 29°C.

Dans les conditions naturelles d'ensemencement, des oeufs d'insectes, pondus sur un milieu de culture comprenant un mélange de tourteaux de palmiste sec et d'eau (1/2 ; poids/poids) connu pour attirer les femelles donnent naissance, 21 jours plus tard à des larves.

De manière remarquable, l'inventeur a pu montrer que ces mini-larves résultent d'un phénomène de diapause provoquée par : (1) la déshydratation du milieu de culture sur lequel elles se développent et, (2) une carence en nourriture consécutive à une surpopulation des larves. La taille des larves est facilement contrôlée par la proportion entre la masse d'oeufs d'insectes et la quantité de milieu disponible.

L'expression « diapause » décrit une forme de vie ralentie et une réaction de survie des larves placées face à des conditions environnementales hostiles. Cette diapause permet d'arrêter la croissance et maintenir les larves dans leur métabolisme basal en attendant la commercialisation.

L'expression « déshydratation » signifie que le milieu de culture est progressivement appauvri en eau jusqu'à comprendre, au maximum, 15% d'eau (poids/poids), idéalement 10% d'eau (poids/poids).

Dans un mode de réalisation avantageux de l'invention, les larves vivantes sont maintenues, avant l'étape de déshydratation à une température de 24 à 35°C, notamment de 27 à 32°C, avantageusement de 29°C, pendant 3 à 6 jours après l'éclosion des oeufs d'insectes, notamment 4 jours.

L'invention a également pour objet l'utilisation des larves d'insectes obtenues selon le procédé de l'invention pour la préparation d'aliments pour poissons d'aquarium ou animaux de compagnie tels que les petits rongeurs, les lézards et les oiseaux. Les larves d'insectes obtenues selon le procédé de l'invention sont particulièrement utiles pour la préparation d'aliments pour alevins et poissons disposant d'un orifice buccal d'un diamètre compris de 1,2 à 4 mm, notamment de 2 à 3 mm.

Les « mini-larves » d'insectes, obtenues par le procédé de la présente invention présentent d'excellentes qualités nutritionnelles et sont très attractives. Les mini-larves sont des larves dont les tailles sont homogènes et qui peuvent être regroupées suivant quatre calibrage du diamètre de leur corps (1,5 mm ; 2,0 mm ; 3,0 mm et 4,0 mm). La production des minilarves selon ces 4 calibrages est assurée par ajustement des proportions entre la quantité d'oeufs mise en culture (masse en milligrammes) et la quantité des tourteaux fermentée mise à disposition.

Les poissons et alevins nourris avec les mini-larves de la présente invention présentent une croissance pondérale nettement plus rapide que lorsqu'ils sont nourris avec d'autres types de proies vivantes disponibles dans le commerce.

La présente invention sera mieux comprise à l'aide des exemples 1 à 7 qui suivent décrivant les procédés de production et l'utilisation de mini-larves d'insecte vivantes pour l'alimentation des poissons d'aquarium et des alevins des poissons d'élevage.

### Exemple 1 - Préparation d'un milieu de culture à base de tourteaux de palmiste pour l'incubation des oeufs et larves de Hermetia illucens

Pour attirer les femelles de *Hermetia illucens* sur un lieu de ponte préférentielle et pour incuber les oeufs obtenus ainsi que les larves de *Hermetia illucens,* un milieu de culture a été préparé soigneusement de la manière suivante :
- à l'aide d'un moulin à café, 1 kg de tourteaux de palmiste est broyé jusqu'à obtenir une poudre très fine,
- cette poudre de tourteaux de palmiste est mélangée à 2 kg (soit 2 litres) d'eau jusqu'à obtenir une sorte de purée parfaitement homogène de tourteaux de palmiste,
- le tout est laissé à fermenter, à l'obscurité ou à l'air libre, à une température de 25 à 30°C, pendant une semaine.

A l'issue, la sorte de purée parfaitement homogène de tourteaux de palmiste ainsi fermentée est prête à être utilisée comme milieu de culture des oeufs et des larves de *Hermetia illucens.*

### Exemples 2 - Milieu de culture à base d'avoine pour l'incubation des oeufs et larves de Hermetia illucens

Pour attirer les femelles de *Hermetia illucens* sur un lieu de ponte préférentielle et pour incuber les oeufs obtenus ainsi que les larves de *Hermetia illucens,* un milieu de culture a été préparé soigneusement de la manière suivante :
- à l'aide d'un moulin à café, 1 kg d'avoine est broyé jusqu'à obtenir une poudre très fine,
- cette poudre d'avoine est mélangée à 2 kg (soit 2 litres) d'eau jusqu'à obtenir une sorte de purée parfaitement homogène d'avoine,
- un pour cent (poids/poids par rapport au poids sec d'avoine) de levure est ajouté à cette sorte de purée parfaitement homogène d'avoine et le tout est mélangé avec soin,
- le tout est laissé à fermenter, à l'obscurité ou à l'air libre, à une température de 25 à 30°C, pendant une semaine.

A l'issue, la sorte de purée parfaitement homogène d'avoine ainsi fermentée est prête à être utilisée comme milieu de culture des oeufs et des larves de *Hermetia illucens.*

### Exemple 3 - Production d'oeufs de Hermetia illucens

Dans une volière adaptée où ils sont nés de 1 kg de biomasse de pupes qui y avaient été déposées dans des bacs en plastique adaptés, mâles et femelles de *Hermetia illucens* peuvent s'accoupler librement et les pontes interviennent une à trois semaines plus tard.

Chaque jour, des sacs en plastique, de type « sacs poubelles sans fond», munis de liens de fermeture intégrés et remplis de milieu de culture selon les Exemples 1 ou 2 sont partiellement fermés par traction sur les liens de fermeture pour créer, au pourtour de l'ouverture desdits sacs en plastique, des plis et des fronces formant entre eux des anfractuosités dans lesquelles les femelles *Hermetia illucens,* attirées par le milieu de culture, viennent pondre préférentiellement.

Tous les 3 jours, 15 à 20 g d'oeufs de *Hermetia illucens* sont ainsi collectés soigneusement par ouverture précautionneuse des susdits sacs en plastique.

Pour éviter toute erreur de pesée, les oeufs doivent être collectés très proprement, sans débris, et sans être accompagnés d'eau ou d'humidité excessive.

### Exemple 4 - Production de mini-larves de Hermetia illucens ayant un diamètre corporel d'environ 1,5 à 2 mm et une longueur d'environ 6 mm

Pour obtenir des mini-larves ayant un diamètre corporel d'environ 1,5 à 2 mm et une longueur d'environ 6 mm, 1 g d'oeufs de *Hermetia illucens* obtenus un même jour selon l'Exemple 3 sont incubés avec 500 g de milieu de culture, tel que préparé aux Exemples 1 ou 2 et, étalés sur 3 à 5 cm d'épaisseur au fond de bacs de culture en fibre de verre. Trois jours plus tard, les éclosions sont observées.

Cinq jours après l'éclosion des oeufs et l'apparition des larves, il est procédé à une ventilation du milieu de culture permettant sa déshydratation progressive jusqu'à atteindre, 2 jours plus tard, une teneur en eau de l'ordre de 10% (poids/poids). Dans de telles conditions de déshydratation, 10 jours après l'ensemencement des oeufs sur le milieu de culture, la croissance des larves est stoppée au stade de maturité voulue.

Les mini-larves ainsi obtenues sont soigneusement nettoyées (sans eau) grâce à un pinceau et placées en diapause (*i.e.* forme de vie ralentie permettant une adaptation de l'organisme à des conditions environnementales hostiles), à l'obscurité, dans un local thermorégulé à une température de 15°C et sous un taux d'hygrométrie de 70%.

Dans de telles conditions, les mini-larves peuvent être maintenues vivantes pendant 4 à 6 semaines et être utilisées comme proies vivantes pour l'alimentation des poissons d'aquarium et des alevins des poissons d'élevage.

Selon les besoins, l'état de diapause peut être interrompu et les mini-larves peuvent être replacées en milieu de culture où leur croissance et leur développement vont alors pouvoir se poursuivre jusqu'à ce qu'elles atteignent, une taille supérieure, à partir de laquelle elles pourront à nouveau être placées en diapause, voire leur taille normale.

### Exemple 5 - Production de mini-larves de Hermetia illucens ayant un diamètre corporel d'environ 3 à 4 mm et une longueur d'environ 6,2 mm

Pour obtenir des mini-larves ayant un diamètre corporel d'environ 2,5 à 3 mm et une longueur d'environ 10 mm, 2 g d'oeufs de *Hermetia illucens* obtenus un même jour selon l'Exemple 3 sont incubés avec 2 kg de milieu de culture, tel que préparé aux Exemples 1 ou 2 et, étalés sur 3 à 5 cm d'épaisseur au fond de bacs de culture en fibre de verre. Trois jours plus tard, les premières éclosions sont observées.

Dix jours après l'éclosion des oeufs et l'apparition des larves, il est procédé à une ventilation du milieu de culture permettant sa déshydratation progressive jusqu'à atteindre, 2 jours plus tard, une teneur en eau de l'ordre de 10% (poids/poids). Dans de telles conditions de déshydratation, 10 jours après l'ensemencement des oeufs sur le milieu de culture, la croissance des larves est stoppée au stade de maturité voulue.

Les mini-larves ainsi obtenues sont soigneusement nettoyées (sans eau) grâce à un pinceau et placées en diapause *(i.e.* forme de vie ralentie permettant une adaptation de l'organisme à des conditions environnementales hostiles), à l'obscurité, dans un local thermorégulé à une température de 15°C et sous un taux d'hygrométrie de 70%.

Dans de telles conditions, les mini-larves peuvent être maintenues vivantes pendant 4 à 6 semaines et être utilisées comme proies vivantes pour l'alimentation des poissons et des alevins.

Selon les besoins, l'état de diapause peut être interrompu et les mini-larves peuvent être replacées en milieu de culture où leur croissance et leur développement vont alors pouvoir se poursuivre jusqu'à ce qu'elles atteignent, une taille supérieure, à partir de laquelle elles pourront à nouveau être placées en diapause, voire leur taille normale.

### Exemple 6 - Effets de la disponibilité du milieu de culture sur l'arrêt de croissance des mini-larves de l'insecte Hermetia illucens

Les conditions d'élevage sont celle de l'exemple 1 mais on fait varier la proportion entre la masse d'oeufs et le milieu de culture de 500 (1g d'oeufs pour 500g de milieu de culture) à 2000 (1 g d'oeufs pour 2 kg de milieu de culture).

Les résultats sur la longueur moyenne, le diamètre moyen corporel et le poids moyen corporel sont donnés dans les tableaux 1A à 1C ci-dessous.

**Tableau 1**

| **A** | | | | |
|---|---|---|---|---|
| Durée de culture (jour) | Longueur moyenne corporelle (mm) | | | |
| | Proportion masse d'oeufs/milieu de culture | | | |
| | 500X | 1000X | 1500X | 2000X |
| J7 | 5,58 | 5,99 | 7,52 | 10,19 |
| J14 | 6,19 | 8,91 | 12,10 | 16,28 |
| J21 | 5,92 | 9,23 | 13,08 | 18,05 |
| | | | | |

| **B** | | | | |
|---|---|---|---|---|
| Durée de culture (jour) | Diamètre moyen corporel (mm) | | | |
| | Proportion masse d'oeufs/milieu de culture | | | |
| | 500X | 1000X | 1500X | 2000X |
| J7 | 1,5 | 1,59 | 2,08 | 3,29 |
| J14 | 1,59 | 2,24 | 3,02 | 4,31 |
| J21 | 1,87 | 2,38 | 3,26 | 4,54 |
| | | | | |

| **C** | | | | |
|---|---|---|---|---|
| Durée de culture (jour) | Poids moyen individuel (mg) | | | |
| | Proportion masse d'oeufs/milieu de culture | | | |
| | 500X | 1000X | 1500X | 2000X |
| J7 | 8,99 | 10,23 | 24,63 | 44,33 |
| J14 | 9,61 | 21,73 | 51,72 | 117,28 |
| J21 | 7,44 | 20,89 | 58,84 | 128,80 |

### Exemple 7 - Valeur nutritive et intérêt des mini-larves de Hermetia illucens par rapport aux autres proies vivantes pouvant constituer l'alimentation des poissons

Des tests ont été réalisés pour démontrer la qualité nutritionnelle et les avantage des mini-larves vis-à-vis des autres proies vivantes pouvant pourvoir à l'alimentation des poissons, notamment les poissons d'aquarium et les alevins des poissons de piscicultures disposant d'un orifice buccal d'un diamètre compris de 1,2 à 3 mm, notamment de 1,8 à 2 mm.

*Chromobotia macracanthus* (Famille des Cobitidés), appelé également le « *Botia* », est un poisson d'ornement très demandé dans le monde de l'aquariophilie. Actuellement, l'approvisionnement en cette espèce sur le marché de l'aquariophilie provient exclusivement de captures réalisées en milieu naturel (Sumatra - Kalimantan).

Cette espèce étant actuellement en voie de disparition, une technique de reproduction des *Botia* en captivité a été récemment mise au point. Toutefois, des difficultés subsistent dans la mesure où l'alimentation des jeunes *Botia* est réputée très difficile avec des aliments inertes types, farines, granulés ou paillettes. Ces poissons et leurs alevins s'alimentent de préférence de proies vivantes. Pour les stades très jeunes (moins d'un mois), le sevrage se fait avec les *nautplii* d'Artémia. Pour les stades plus avancés, entre 1 et 3 mois, on utilise des lombrics coupés ou des vers de vase (« blood worms »). Ces derniers sont des larves de l'insecte Chironome collectées, dans la vase, au fond de mares ou de marigots.

L'utilisation de ces proies vivantes pose toutefois un problème d'hygiène et de salubrité dans la mesure où elles sont bien souvent porteuses de germes pathogènes susceptibles d'infecter les poissons et alevins. Il est donc nécessaire de rechercher d'autres types de proies vivantes qui ne présenteront pas cet inconvénient et qui auront, au moins, les mêmes qualités nutritionnelles que les lombrics ou les vers de vase.

Pendant une période de 70 jours, des mini-larves produites selon l'Exemple 4 et ayant un diamètre corporel d'environ 1,5 mm et une longueur d'environ 6 mm, des lombrics coupés ou des vers de vase ont été proposés, dans des conditions tout à fait identiques, à de jeunes alevins de *Botia* de 2 mois pesant environ 0,23 g.

Au cours de cette période de 70 jours, il a été observé que la croissance et le développement des alevins nourris avec les mini-larves de *Hermetia illucens* est 1,2 fois plus rapide que ceux nourris avec les vers de vase et 2 fois plus rapide que ceux nourris avec des lombrics coupés.

## Revendications

1. Procédé d'obtention de larves d'insectes conservées vivantes, dont le diamètre maximal, de 1 à 4 mm, notamment de 2 mm environ et dont la longueur corporelle maximale de 4 à 12 mm, notamment de 5 à 12 mm, notamment de 4 à 8 mm, notamment de 9 mm, notamment de 6 mm, sont maintenus sensiblement constants pendant au moins 4 semaines, notamment 6 semaines, ledit procédé comprenant les étapes suivantes :
1) incubation d'oeufs d'insectes dans un milieu de culture comprenant un mélange d'eau, de céréales et/ou de tourteaux et, éventuellement, d'un agent de fermentation, ladite incubation étant conduite jusqu'à l'éclosion des oeufs et l'obtention des larves, pour obtenir une composition comprenant un milieu de culture, lequel comprend un mélange d'eau, de céréales et/ou de tourteaux et, éventuellement, un agent de fermentation et des oeufs et/ou des larves d'insectes incubés dans ledit milieu de culture.
2) déshydratation de la susdite composition jusqu'à obtenir une teneur en eau inférieure à 15% (poids/poids), idéalement de 10% (poids/poids),
3) collecte, à partir de la susdite composition, des larves d'insectes vivantes, de diamètre corporel maximal de 1 à 4 mm, notamment de 2 mm environ et de longueur corporelle maximale de 4 à 12 mm, notamment de 5 à 12 mm, notamment de 4 à 8 mm, notamment de 9 mm, notamment de 6 mm, et,
4) transfert des susdites larves vivantes collectées dans un environnement dépourvu de nourriture où elles sont maintenues vivantes, à l'obscurité, pendant au moins 4 semaines, notamment 6 semaines, sous un taux d'hygrométrie de 60 à 80% et à une température de 12 à 18°C, notamment 15°C.

2. Procédé selon la revendication 1 **caractérisé en ce que** les oeufs d'insectes sont obtenus à partir de populations d'insectes confinées dans une volière les isolant totalement de l'extérieur et les protégeant des prédations par d'autres insectes ou par des animaux, ladite volière étant aménagée de telle manière à reproduire l'habitat naturel des insectes qui y sont maintenus.

3. Procédé selon l'une quelconque des revendications 1 ou 2 **caractérisé en ce que** les insectes appartiennent au groupe des holométaboles en particulier, *Hermetia illucens,* élevé à l'intérieur d'une volière dans une atmosphère dont le degré d'hygrométrie et la température sont maintenus respectivement de 70 à 80% et de 27 à 30°C, avantageusement en présence de végétaux du type *Sphagneticola sp.*

4. Procédé selon l'une quelconque des revendications 1 à 3 **caractérisé en ce que** les pupes d'insectes sont placées à l'intérieur de la volière dans des bacs d'exuviation en plastique à raison d'environ 5 000 à 10 000 pupes par bac en plastique.

5. Procédé selon l'une quelconque des revendications 1 à 4 **caractérisé en ce que** les oeufs d'insectes sont pondus dans des anfractuosités artificielles spécialement créées à cet effet à proximité immédiate d'un milieu de culture.

6. Procédé selon l'une quelconque des revendications 1 à 5 dans lequel les céréales et/ou les tourteaux sont soumis à une fermentation, à l'obscurité ou à l'air libre, pendant 1 semaine et à une température de 27 à 40°C, notamment de 27 à 32°C.

7. Procédé selon l'une quelconque des revendications 1 à 6 dans lequel la proportion pondérale eau/céréales ou eau/tourteaux dans le milieu de culture est de 1 à 3, notamment de 1 à 2,2 et, est, de préférence, égale à 2.

8. Procédé selon l'une quelconque des revendications 1 à 7, dans lequel la proportion pondérale eau/céréales+tourteaux est de 1 à 3, de préférence, égale à 2.

9. Procédé selon l'une quelconque des revendications 1 à 8, dans lequel le milieu de culture comprend un agent de fermentation ajouté à raison de 0 à 2% en poids par rapport au poids sec de céréales et/ou de tourteaux.

10. Procédé selon l'une quelconque des revendications 1 à 9, dans lequel le milieu de culture comprend des tourteaux et est totalement dépourvu de tout agent de fermentation ajouté.

11. Procédé selon l'une quelconque des revendications 1 à 10, dans lequel le milieu de culture comprend un agent de fermentation choisi parmi les bactéries, les champignons microscopiques choisis dans le groupe comprenant des levures.

12. Procédé selon l'une quelconque des revendications 1 à 11, dans lequel le milieu de culture comprend des céréales, lesdites céréales étant choisies parmi le blé, l'épeautre, le maïs, l'orge, l'escourgeon, le riz, le millet, le seigle, le sorgho et l'avoine.

13. Procédé selon l'une quelconque des revendications 1 à 11, dans lequel le milieu de culture comprend des tourteaux, lesdits tourteaux étant choisis parmi les tourteaux de soja, d'arachide, de colza, de tournesol, de lin, de pépin de raisin, d'olive, de copra et de palmiste.

14. Procédé selon l'une quelconque des revendications 1 à 13, dans lequel l'étape d'incubation et d'éclosion des oeufs d'insectes dans le milieu de culture est effectuée pendant 1 à 3 jours, notamment 2 jours, à une température de 24 à 35°C, notamment de 27 à 32°C, idéalement de 29°C.

15. Procédé selon l'une quelconque des revendications 1 à 14 dans lequel les larves vivantes sont maintenues, avant l'étape de déshydratation à une température de 24 à 35°C, notamment de 27 à 32°C, idéalement de 29°C, pendant 3 à 6 jours après l'éclosion des oeufs d'insectes, notamment 4 jours.

16. Utilisation des larves d'insectes obtenues selon l'une quelconque des revendications 1 à 15, pour la préparation d'aliments pour poissons d'aquarium et animaux de compagnie.

17. Utilisation des larves d'insectes selon la revendication 16, pour la préparation d'aliments pour alevins et poissons disposant d'un orifice buccal d'un diamètre compris de 1,2 à 4 mm, notamment de 2 à 3 mm.

18. Composition comprenant un milieu de culture, lequel comprend un mélange d'eau, de céréales et/ou de tourteaux et, éventuellement, un agent de fermentation, des oeufs incubés dans ledit milieu de culture à raison de 0,5 à 1,5 g/kg de milieu de culture, notamment 1 g/kg de milieu de culture, les céréales et/ou les tourteaux ayant été soumis à une fermentation, à l'obscurité ou à l'air libre, pendant 1 semaine et à une température de 27 à 40°C, notamment de 27 à 32°C, ladite composition comprenant également des larves d'insectes incubés dans ledit milieu de culture ayant un diamètre corporel maximal de 1 à 4 mm, notamment de 2 mm et une longueur corporelle maximale de 4 à 12 mm, notamment de 5 à 12 mm, notamment de 4 à 8 mm, notamment de 9 mm, notamment de 6 mm.

19. Composition selon la revendication 18 **caractérisée en ce que** les oeufs et/ou les larves sont issus de *Hermetia illucens.*

## Patentansprüche

1. Verfahren zum Erhalten von lebend konservierten Insektenlarven, deren maximaler Durchmesser 1 bis 4 mm, insbesondere etwa 2 mm, beträgt und deren maximale Körperlänge 4 bis 12 mm, insbesondere 5 bis 12 mm, insbesondere 4 bis 8 mm, insbesondere 9 mm, insbesondere 6 mm, beträgt, die für mindestens 4 Wochen, insbesondere 6 Wochen, im Wesentlichen konstant gehalten werden, wobei das Verfahren die folgenden Schritte umfasst:
1) Inkubieren von Insekteneiern in einem Kulturmedium, das ein Gemisch aus Wasser, Getreide und/oder Presskuchen und gegebenenfalls ein Fermentationsmittel umfasst, wobei die Inkubation bis zum Schlüpfen der Eier und zum Erhalten der Larven durchgeführt wird, um eine Zusammensetzung zu erhalten, die ein Kulturmedium umfasst, das ein Gemisch aus Wasser, Getreide und/oder Presskuchen und gegebenenfalls ein Fermentationsmittel und in dem Kulturmedium inkubierte Insekteneier und/oder Insektenlarven umfasst,
2) Dehydrieren dieser Zusammensetzung bis zum Erhalten eines Wassergehalts von weniger als 15 % (Gew./Gew.), idealerweise 10 % (Gew./Gew.),
3) Gewinnen lebender Insektenlarven mit einem maximalen Körperdurchmesser von 1 bis 4 mm, insbesondere etwa 2 mm, und mit einer maximalen Körperlänge von 4 bis 12 mm, insbesondere 5 bis 12 mm, insbesondere 4 bis 8 mm, insbesondere 9 mm, insbesondere 6 mm, aus dieser Zusammensetzung, und
4) Übertragen dieser gewonnenen, lebenden Larven in eine Umgebung, die frei von Nahrung ist, in der sie im Dunkeln für mindestens 4 Wochen, insbesondere 6 Wochen, bei einer Luftfeuchtigkeit von 60 bis 80 % und bei einer Temperatur von 12 bis 18 °C, insbesondere 15 °C, lebend gehalten werden.

2. Verfahren nach Anspruch 1, **dadurch gekennzeichnet, dass** die Insekteneier aus Insektenpopulationen erhalten werden, die in einem Käfig eingeschlossen sind, der sie vollständig von der Außenwelt isoliert und sie vor Prädationen durch andere Insekten oder durch Tiere schützt, wobei der Käfig derart ausgestattet ist, dass er das natürliche Habitat der Insekten, die dort gehalten werden, reproduziert.

3. Verfahren nach einem der Ansprüche 1 oder 2, **dadurch gekennzeichnet, dass** die Insekten zur Gruppe der holometabolen Insekten, insbesondere Hermetia illucens, gehören, die im Innern eines Käfigs in einer Atmosphäre gezüchtet werden, deren Luftfeuchtigkeit und Temperatur auf 70 bis 80 % bzw. 27 bis 30 °C gehalten werden, vorteilhafterweise in Gegenwart von Pflanzen des Typs Sphagneticola sp.

4. Verfahren nach einem der Ansprüche 1 bis 3, **dadurch gekennzeichnet, dass** die Insektenpuppen im Innern des Käfigs in Häutungstanks aus Kunststoff in einer Menge von etwa 5.000 bis 10.000 Puppen je Kunststofftank angeordnet werden.

5. Verfahren nach einem der Ansprüche 1 bis 4, **dadurch gekennzeichnet, dass** die Insekteneier in künstliche Vertiefungen gelegt werden, die speziell zu diesem Zweck in unmittelbarer Nähe eines Kulturmediums erzeugt wurden.

6. Verfahren nach einem der Ansprüche 1 bis 5, wobei das Getreide und/oder die Presskuchen im Dunkeln oder im Freien 1 Woche lang und bei einer Temperatur von 27 bis 40 °C, insbesondere 27 bis 32 °C einer Fermentation unterzogen werden.

7. Verfahren nach einem der Ansprüche 1 bis 6, wobei der Gewichtsanteil Wasser/Getreide oder Wasser/Presskuchen in dem Kulturmedium 1 bis 3, insbesondere 1 bis 2,2 und bevorzugt gleich 2 ist.

8. Verfahren nach einem der Ansprüche 1 bis 7, wobei der Gewichtsanteil Wasser/Getreide und Presskuchen 1 bis 3, bevorzugt gleich 2 ist.

9. Verfahren nach einem der Ansprüche 1 bis 8, wobei das Kulturmedium ein Fermentationsmittel umfasst, das in einer Menge von 0 bis 2 Gew.-%, bezogen auf das Trockengewicht des Getreides und/oder der Presskuchen, zugegeben wird.

10. Verfahren nach einem der Ansprüche 1 bis 9, wobei das Kulturmedium Presskuchen umfasst und vollständig frei von jedem zugegebenen Fermentationsmittel ist.

11. Verfahren nach einem der Ansprüche 1 bis 10, wobei das Kulturmedium ein Fermentationsmittel umfasst, ausgewählt aus Bakterien, mikroskopischen Pilzen, ausgewählt aus der Gruppe, die Hefen umfasst.

12. Verfahren nach einem der Ansprüche 1 bis 11, wobei das Kulturmedium Getreide umfasst, wobei das Getreide ausgewählt ist aus Weizen, Dinkel, Mais, Gerste, Wintergerste, Reis, Hirse, Roggen, Sorghum und Hafer.

13. Verfahren nach einem der Ansprüche 1 bis 11, wobei das Kulturmedium Presskuchen umfasst, wobei die Presskuchen ausgewählt sind aus Sojakuchen, Erdnusskuchen, Rapskuchen, Sonnenblumenkuchen, Leinkuchen, Traubenkernkuchen, Olivenkuchen, Koprakuchen und Palmkernkuchen.

14. Verfahren nach einem der Ansprüche 1 bis 13, wobei der Schritt zur Inkubation und zum Schlüpfen der Insekteneier in dem Kulturmedium für 1 bis 3 Tage, insbesondere 2 Tage, bei einer Temperatur von 24 bis 35 °C, insbesondere 27 bis 32 °C, idealerweise von 29 °C durchgeführt wird.

15. Verfahren nach einem der Ansprüche 1 bis 14, wobei die lebenden Larven vor dem Schritt zur Dehydrierung bei einer Temperatur von 24 bis 35 °C, insbesondere 27 bis 32 °C, idealerweise von 29 °C, für 3 bis 6 Tage, insbesondere für 4 Tage, nach dem Schlüpfen der Insekteneier erhalten werden.

16. Verwendung der gemäß einem der Ansprüche 1 bis 15 erhaltenen Insektenlarven zur Herstellung von Nahrungsmitteln für Aquariumsfische und Haustiere.

17. Verwendung der Insektenlarven nach Anspruch 16 zur Herstellung von Nahrungsmitteln für Setzlinge und Fische, die über eine Mundöffnung mit einem Durchmesser im Bereich von 1,2 bis 4 mm, insbesondere 2 bis 3 mm verfügen.

18. Zusammensetzung, die ein Kulturmedium umfasst, das ein Gemisch aus Wasser, Getreide und/oder Presskuchen und gegebenenfalls ein Fermentationsmittel, in dem Kulturmedium inkubierte Eier in einer Menge von 0,5 bis 1,5 g/kg Kulturmedium, insbesondere 1 g/kg Kulturmedium umfasst, wobei das Getreide und/oder die Presskuchen einer Fermentation im Dunkeln oder im Freien für 1 Woche und bei einer Temperatur von 27 bis 40 °C, insbesondere von 27 bis 32 °C, unterzogen wurden, wobei die Zusammensetzung auch in dem Kulturmedium inkubierte Insektenlarven umfasst, die einen maximalen Körperdurchmesser von 1 bis 4 mm, insbesondere von 2 mm, und eine maximale Körperlänge von 4 bis 12 mm, insbesondere von 5 bis 12 mm, insbesondere von 4 bis 8 mm, insbesondere von 9 mm, insbesondere von 6 mm, aufweisen.

19. Zusammensetzung nach Anspruch 18, die Eier und/oder Larven umfasst, die von Hermetia illucens stammen.

## Claims

1. Process for obtaining insect larvae kept alive, whose maximum diameter, from 1 to 4 mm, notably about 2 mm, and whose maximum body lengths, from 4 to 12 mm, notably from 5 to 12 mm, notably from 4 to 8 mm, notably 9 mm, notably 6 mm, are maintained sensitively constant during at least 4 weeks, notably 6 weeks, said process comprising the following steps :
1) incubation of insect eggs in a culture medium comprising a mixture of water, cereals and/or oil cakes, and possibly a fermentation agent, said incubation being carried out until the hatching of eggs and obtaining of larvae, in order to obtain a composition comprising a culture medium, said medium comprising a mixture of water, cereals and/or oil cakes, and possibly a fermentation agent and insect eggs and/or larvae incubated in said culture medium.
2) dehydration of said composition until obtaining a water content below 15% (weight/weight), ideally 10% (weight/weight),
3) collection, from said composition, of living insect larvae, with a maximum body diameter from 1 to 4 mm, notably about 2 mm, and a maximum body length from 5 to 12, notably from 4 to 8 mm, notably 9 mm, notably 6 mm, and,
4) transfer of said collected living larvae in an environment without feed, where they are maintained alive, in the dark, during at least 4 weeks, notably 6 weeks, under a hygrometry rate from 60 to 80% and at a temperature from 12 to 18°C, notably 15°C.

2. Process according to claim 1 **characterised in that** the eggs of insect are obtained from insect populations confined in an aviary which isolates them totally from the outside environment and protects them from predation by other insects or by animals, said aviary being arranged in such a manner in order to reproduce the natural habitat cf insects which are kept there.

3. Process according to any of claim 1 or claim 2 **characterised in that** the insects belong to the holometabolic group, in particular *Hermetia illucens,* reared inside an aviary in an atmosphere whose hygrometry rate and temperature are maintained respectively from 70 to 80% and from 27 to 30°C, advantageously in presence of plants belonging to *Sphagneticola sp.*

4. Process according to any of claims 1 to 3 **characterised in that** insect pupae are placed inside the aviary within plastic exuviation trays for about 5 000 to 10 000 pupae per plastic tray.

5. Process according to any of claims 1 to 4 **characterised in that** insect eggs are laid in artificial crevices specially created to that effect in the immediate vicinity of a culture medium.

6. Process according to any of claims 1 to 5 in which the cereals and/or oil cakes are subject to a fermentation, in the dark or in free air, during 1 week and at a temperature from 27 to 40°C, notably from 27 to 32°C.

7. Process according to any of claims 1 to 6, in which the weight proportion of water/cereals or water/oil cakes in the culture medium is from 1 to 3, notably from 1 to 2.2 and, is preferably equal to 2.

8. Process according to any of claims 1 to 7, in which the weight proportion of the water/cereals+oil cakes is from 1 to 3, preferably equal to 2.

9. Process according to any of claims 1 to 8, in which the culture medium comprises a fermentation agent added at a rate from 0 to 2 % by weight in relation to the dry weight of the cereals and/or oil cakes.

10. Process according to any of claims 1 to 9, in which the culture medium comprises oil cakes, and is totally without of any added fermentation agent.

11. Process according to any of claims 1 to 10, in which the culture medium comprises a fermentation agent chosen from among bacteria, microscopic fungi as chosen within the group comprising yeasts.

12. Process according to any of claims 1 to 11, in which the culture medium comprises cereals, said cereals being chosen from among wheat, spelt, maize, barley, winter barley, rice, millet, rye, sorghum and oats.

13. Process according to any of claims 1 to 11, in which the culture medium comprises oil cakes, said oil cakes being chosen from among oil cakes of soy, groundnuts, rape, sunflower, flax, grape pip, olive, sorgho and palm kernel.

14. Process according to any of claims 1 to 13, in which the insect egg incubation and hatching step in the culture medium is carried out during 1 to 3 days, notably 2 days, at a temperature from 24 to 35°C, notably from 27 to 32°C, ideally 29°C.

15. Process according to any of claims 1 to 14, in which the living larvae are maintained, before the dehydration step, at a temperature from 24 to 35°C, notably from 27 to 32°C, ideally 29°C, during 3 to 6 days after insect egg hatching, notably 4 days.

16. Use of insect larvae as obtained according to any of claims 1 to 15, for the preparation of aquarium fish and domestic animal feed.

17. Use of insect larvae as obtained according to claims 16, for the preparation of a feed for fry and fishes with an oral diameter comprised from 1.2 to 4 mm, notably from 2 to 3 mm.

18. Composition comprising a culture medium, which includes a mixture of water, cereals and/or oil cakes and, possibly a fermentation agent, incubated eggs in said culture medium at a rate of 0.5 to 1.5 g/kg culture medium, notably 1 g/kg culture medium, cereals and/or oil cakes having been subject to a fermentation, in the dark or in free air, during 1 week and at a temperature from 27 to 40°C, notably from 27 to 32°C, said composition also comprising insect larvae, incubated in said culture medium, having a maximum body diameter from 1 to 4 mm, notably 2 mm, and a maximum body length from 4 to 12, notably from 5 to 12, notably from 4 to 8 mm, notably 9 mm, notably 6 mm.

19. Composition according to claim 18, **characterised in that** the eggs and/or larvae come from *Hermetia illucens.*
